# EUROPÄISCHE PATENTSCHRIFT

(11) **EP 1 391 190 B1**
(45) Veröffentlichungstag und Bekanntmachung des Hinweises auf die Patenterteilung: **16.11.2005**
(21) Anmeldenummer: 03017187.0
(22) Anmeldetag: 29.07.2003
(51) Int. Cl.: A61F 13/08, A61F 13/06

(54) **Kompressionsstrumpf mit Kompressionskörper**
Elastic stockings with compression insert
Jambière à compression avec insert de compression

(30) Priorität: 12.08.2002 DE 10237374
(43) Veröffentlichungstag der Anmeldung: 25.02.2004
(73) Patentinhaber: Schneider, Thomas, 99817 Eisenach (DE); Schindewolf, Ulrich, 99817 Eisenach (DE); Härer, Thomas, Dr. med., 36251 Bad Hersfeld (DE)
(72) Erfinder: Schneider, Thomas, 99817 Eisenach (DE); Schindewolf, Ulrich, 99817 Eisenach (DE); Härer, Thomas, Dr. med., 36251 Bad Hersfeld (DE)
(74) Vertreter: Scheffler, Jörg

(56) Entgegenhaltungen:
- EP-A- 0 027 172
- EP-A- 0 262 638
- EP-B- 0 598 291
- DE-A- 3 838 576
- DE-A- 4 341 722
- DE-A- 19 506 128
- DE-U- 1 929 624
- US-A- 4 700 698

## Beschreibung

Die Erfindung betrifft einen Kompressionsstrumpf aus einem elastischen Gewebe, Gewirke oder Gestricke mit einem an dem Kompressionsstrumpf fixierbaren Kompressionskörper aus einem elastischen Material.

Kompressionsstrümpfe an sich sind in der Praxis hinlänglich bekannt, bestehen im wesentlichen aus einem elastischen Gewebe, Gewirke oder Gestricke und verlaufen über die gesamte Länge des Beines. Kompressionsstrümpfe werden unter anderem bei venösen Erkrankungen, beispielsweise bei Auftreten von Krampfadern, eingesetzt. Durch den Kompressionsstrumpf wird eine regelmäßige Beschleunigung des venösen Blutstroms zum Herzen erreicht und venöse Stauungen werden beseitigt. Die krankhafte Fehlfunktion der Venenklappen wird durch die Kompression von außen korrigiert, weil die Pumpwirkung der Wadenmuskelpumpe wieder hergestellt wird. Weiterhin wird verhindert, dass verbrauchtes Blut und Gewebeflüssigkeit sich im Gewebe ansammeln und stauen, so dass das Gewebe vor den Schäden eines zunehmenden Ödems geschützt wird. Somit kann der Kompressionsstrumpf auch in der Thromboseprophylaxe, beispielsweise nach einem operativen Eingriff, eingesetzt werden.

Ein Kompressionsstrumpf der eingangs genannten Art ist aus der DE 195 06 128 A1 bekannt. Hierin wird ein Verfahren zum Fixieren von Kompressionskörpern an Kompressionsstrümpfen beschrieben. Die Kompressionskörper sind als elastische Druckpolster oder schützende und stützende Polster ausgebildet. Auf die genaue Ausgestaltung, den Befestigungsort und die Wirkungsweise der Polster bzw. Pelotten und den Anwendungsbereich des Kompressionsstrumpfes wird allerdings nicht weiter eingegangen.

Weiterhin ist aus der DE 43 41 722 A1 ein Kompressionsstrumpf mit einem integrierten, als Abpolsterungseinlage ausgebildeten Kompressionskörper zur Behandlung von lymphostatischen Fibrosen bekannt. Die Abpolsterung wird von einem flächigen, elastischen Schaumkörper gebildet und weist eine Vielzahl von Erhebungen und Vertiefungen auf. Bei Bewegungen des entsprechenden Körperteils üben diese Erhebungen und Vertiefungen einen mechanischen Reiz auf das erkrankte Gewebe aus, wodurch das Abströmen der angestauten Gewebsflüssigkeit auch unter krankhaft veränderten Bedingungen ermöglicht bzw. verbessert wird.

Dabei hat es sich als nachteilig erwiesen, dass das Schaummaterial in Folge seiner hohen Dichte und der benötigten Materialdicke, immer das Bestreben hat, seine Ursprungsform einzunehmen. Wird also das Schaummaterial aufgrund des umgebenden Kompressionsstrumpfes an die zu komprimierende Struktur gedrückt, so wird durch das Schaummaterial eine Rückstellkraft gebildet, die der Bewegung des entsprechenden Körperteils entgegenwirkt. Die Bewegungsfreiheit des Patienten wird hierdurch erheblich reduziert.

Aus der EP 00 27 172 A1 und der EP 02 62 638 A2 ist jeweils eine schlauchförmige Bandage zur Abstützung bzw. Kompression von Gelenken bekannt. Diese schlauchförmige Bandage wird bei einer Gelenkverletzung über das betreffende Gelenk gezogen und weist eine die Knochenvorsprünge des Gelenks umgebende, ringförmige Kompressionseinlage auf. Diese Kompressionseinlage übt einen Druck auf die Gelenkweichteile aus und erzeugt dadurch einen mechanischen Reiz, der ein schnelleres Abschwellen des verletzten Gelenkes bzw. der Gelenkweichteile bewirkt. Diese Bandagen werden nach Verletzungen oder bei degenerativen Erkrankungen, beispielsweise bei einer Meniskusschädigung, bei Arthrose, Ergüssen und bei Bandverletzungen des Kniegelenks eingesetzt. Der Druck durch die Bandage wird ausschließlich auf die Kompressionseinlage und damit ausschließlich auf die Gelenkweichteile ausgeübt.

Der Erfindung liegt die Aufgabe zugrunde, die Wirkungsweise des Kompressionsstrumpfes zu verbessern und dabei den Anwendungsbereich zu erweitern.

Diese Aufgabe wird erfindungsgemäß mit einem Kompressionsstrumpf gemäß den Merkmalen des Patentanspruchs 1 gelöst. Die weitere Ausgestaltung der Erfindung ist den Unteransprüchen 2 bis 9 zu entnehmen.

Erfindungsgemäß ist also ein Kompressionsstrumpf, bei dem der Kompressionskörper eine Aussparung zur Aufnahme einer Kniescheibe eines Kniegelenks aufweist, wobei die benachbarten Gelenkweichteile mittels des die Aussparung einschließenden Kompressionskörpers mit Druck beaufschlagbar sind und bei dem eine den Gelenkweichteilen zugewandte Fläche des Kompressionskörpers zumindest eine nutenförmige Ausnehmung aufweist. Hierdurch wird eine Möglichkeit geschaffen, den erfindungsgemäßen Kompressionsstrumpf auch im unmittelbaren Anschluss an einen operativen Einsatz im Bereich des Kniegelenks einzusetzen. Das nach dem Stand der Technik erforderliche und überaus umständliche Wickeln mittels elastischer Binden zur Druckbeaufschlagung des Kniegelenks und des Beines ist somit nicht mehr erforderlich. Durch den erfindungsgemäßen Kompressionsstrumpf wird gleichzeitig sowohl auf das eingeschlossene Bein, als auch auf den Kompressionskörper ein definierter Druck ausgeübt. Die Kniescheibe des Kniegelenks wir durch die Aussparung des Kompressionskörpers nicht mit einer Druckkraft beaufschlagt. Stattdessen wird der Druck vollständig in die der Kniescheibe benachbarten Gelenkweichteile eingeleitet. Hierdurch wird nach der Operation entstehendes Wundsekret, Blut- und Gewebeflüssigkeit über den venösen Rückfluss aus den Gelenkweichteilen bzw. der Gefäßhülle des Kniegelenks herausgedrückt. Eine Schwellung des Kniegelenks sowie Vereiterungen der Wundnaht werden somit verhindert. Der von dem Kompressionsstrumpf auf die benachbarten Bereiche des Beines ausgeübte Druck verhindert ein Ansammeln und Anstauen dieser Flüssigkeiten im angrenzenden Beinbereich, unterstützt hier den venösen Rückfluss und verhindert somit eine drohende Thrombose. Die durch die Operation hervorgerufene Wundnaht wird zusammen mit den zugeordneten Wundklammern von der Ausnehmung in dem Kompressionskörper aufgenommen. Somit wird die äußerst schmerzempfindliche Wundnaht nicht von dem Druck des Kompressionskörpers beaufschlagt, was wiederum die Wundheilung verbessert, Vereiterungen verhindert und gleichzeitig die Schmerzen des Patienten lindert. Außerdem wird durch die Aufnahme der Wundnaht bzw. der Wundklammern in den Kompressionskörper und das Anordnen der Kniescheibe in der Aussparung des Kompressionskörpers erreicht, dass der Kompressionskörper flächig auf den Bereichen des Gelenks aufliegt, in denen sich die Gelenkweichteile befinden. Somit kann der gesamte von dem Kompressionsstrumpf ausgeübte Druck über den Kompressionskörper auf die Gelenkweichteile übertragen werden.

Eine besonders zweckmäßige Abwandlung der Erfindung wird dadurch geschaffen, dass der Kompressionskörper eine elliptische Form mit einer Hauptachse und einer Nebenachse aufweist. Hierdurch wird der Kompressionskörper an die Form der Gelenkweichteile des Kniegelenks bzw. der Gefäßhülle angepasst, die in etwa die Form einer Ellipse aufweisen. Dabei wird der Kompressionskörper derart ausgerichtet, dass die Hauptachse des Kompressionskörpers in etwa parallel zu einer Körperlängsachse angeordnet ist. Durch die Anpassung des Kompressionskörpers an die Gelenkweichteile wird gewährleistet, dass der gesamte, durch den Kompressionsstrumpf aufgebrachte Druck vollständig und nahezu verlustfrei in die Gelenkweichteile eingeleitet wird.

Dabei erweist es sich als besonders wirkungsvoll, dass die nutenförmige Ausnehmung entlang der Hauptachse des Kompressionskörpers angeordnet ist. Hierdurch ist die nutenförmige Ausnehmung im Kompressionskörper dem bei einem Knieeingriff charakteristischen Nahtverlauf angepasst.

Besonders praxisnah erweist es sich dabei, dass die nutenförmige Ausnehmung eine Breite von etwa 12 bis 20 mm und eine Tiefe von etwa 4 bis 10 mm aufweist. Diese Abmessungen der Ausnehmung haben sich in der Praxis als ausreichend für die Aufnahme der Wundnaht und auch für die Aufnahme der gängigen Größen von Wundklammern erwiesen.

Eine weitere besonders vorteilhafte Ausführungsform wird dadurch geschaffen, dass die Aussparung in dem Kompressionskörper ellipsenförmig ausgebildet ist. Die Form der Aussparung des Kompressionskörpers ist hierbei in etwa an die Form der Kniescheibe angepasst. Hierdurch wird die Einleitung des vom Kompressionskörper ausgeübten Drucks an die benachbarten Gelenkweichteile verbessert und die Kniescheibe wird abgestützt und geführt.

Als besonders praxisnah hat es sich erwiesen, dass die Aussparung bezogen auf eine Hauptachse eine Länge von etwa 70 bis 85 mm und bezogen auf eine Nebenachse eine Breite von etwa 50 bis 60 mm aufweist. Dieser Größenbereich entspricht in etwa der Größe einer Kniescheibe eines Kniegelenks. Vorteilhaft wäre ein Vermessen der Kniescheibe des Patienten vor der Operation, um eine genaue Anpassung der Aussparung im Kompressionskörper an die jeweilige Kniescheibe vornehmen zu können. Hierdurch wird die Druckeinleitung in benachbarte Gelenkweichteile und die Stützwirkung der Kniescheibe weiter verbessert.

Eine andere besonders zweckmäßige Weiterbildung der Erfindung wird dadurch erreicht, dass der Kompressionskörper mittels einer Adhäsionsverbindung lösbar an dem Kompressionsstrumpf fixiert ist. Die Adhäsionsverbindung garantiert eine sichere Verbindung zwischen dem Kompressionskörper und dem Kompressionsstrumpf mit einer minimalen Faltenbildung. Hierdurch entsteht eine glatte Fläche im Berührungsbereich zum Kniegelenk, wodurch wiederum Druckstellen vermieden werden können. Durch die lösbare Fixierung des Kompressionskörpers wird die Flexibilität des Kompressionsstrumpfes zusätzlich erhöht, da der Kompressionsstrumpf einerseits mit fixiertem Kompressionskörper im postoperativen Einsatz, und andererseits auch ohne den Kompressionskörper, beispielsweise ausschließlich zur Thromboseprophylaxe, einsetzbar ist. Außerdem wird hierdurch der Einsatz von unterschiedlichen Kompressionskörpem, beispielsweise für andere Anwendungen in ein und demselben Kompressionsstrumpf ermöglicht.

Dabei erweist es sich als besonders vorteilhaft, dass der Kompressionskörper zumindest abschnittsweise aus Silikon besteht. Silikon zeichnet sich durch eine gute Druckfestigkeit aus und ist deshalb als Material für den Einsatz als Kompressionskörper besonders geeignet. Weiterhin ist eine Desinfizierung von Silikon sehr einfach, was wiederum bei einem Einsatz mit direktem Kontakt zu einer Wundnaht besonders vorteilhaft ist, um beispielsweise Vereiterungen und Entzündungen der Wundnaht zu verhindern. Zudem hat sich Silikon in der Praxis als besonders hautverträglich ausgezeichnet.

Eine andere zweckmäßige Weiterbildung der Erfindung wird dadurch geschaffen, dass zwischen dem Kompressionskörper und den Gelenkweichteilen eine Schutzschicht angeordnet ist. Diese Schutzschicht verhindert einen direkten Kontakt zwischen dem Kompressionskörper und den Gelenkweichteilen und ist insbesondere bei einer Silikonallergie des Patienten besonders vorteilhaft. Weiterhin saugt die Schutzschicht auch aus der Wundnaht austretendes Sekret auf und verbessert dadurch die Wundheilung.

Die Erfindung lässt verschiedene Ausführungsformen zu. Zur weiteren Verdeutlichung ihres Grundprinzips ist eine davon in der Zeichnung dargestellt und wird nachfolgend beschrieben. Diese zeigt in
- Fig.1: eine schematische Vorderansicht einer Person von der Hüfte abwärts, die einen findungsgemäßen Kompressionsstrumpf trägt;
- Fig.2: eine Rückansicht eines Kompressionskörpers nach Fig. 1 in einer vergrößerten und teilweise geschnittenen Darstellung;
- Fig.3: den Kompressionskörper nach Ansicht A aus Fig. 2.

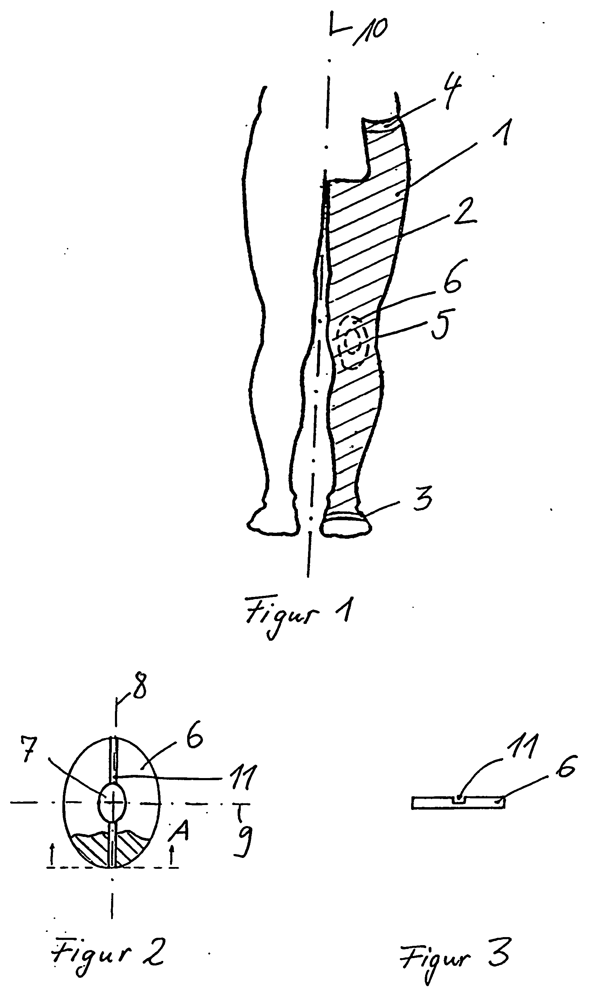

Figur 1 zeigt eine schematische Vorderansicht einer Person von der Hüfte abwärts, die einen erfindungsgemäßen Kompressionsstrumpf 1 trägt. Der Kompressionsstrumpf 1 bedeckt dabei das gesamte Bein 2 vom Fußbereich 3 bis hinauf in den Bereich der Leiste 4. Der Kompressionsstrumpf besteht aus einem elastischen Gewebe, Gewirke oder Gestricke und setzt sich je nach gewünschtem Kompressionsdruck aus unterschiedlichen Materialkomponenten zusammen. Der Kompressionsstrumpf 1 besteht beispielsweise aus 65 % Polyamid und 35 % Elasthan und ist der Kompressionsklasse II zugeordnet. Dabei sind allerdings auch andere Materialzusammensetzungen und Kompressionsklassen des Kompressionsstrumpfes 1 denkbar. Im Bereich eines Kniegelenkes 5 ist zwischen dem Kompressionsstrumpf 1 und dem Kniegelenk 5 ein als Pelotte ausgebildeter Kompressionskörper 6 angeordnet, der an der Innenseite des Kompressionsstrumpfes 1 fixiert ist. Der Kompressionskörper 6 ist in der Figur 1 mit gestrichelten Linien dargestellt, da er unterhalb des Kompressionsstrumpfes 1 angeordnet ist. Der erfindungsgemäße Kompressionsstrumpf 1 kann vor allem im postoperativen Einsatz, beispielsweise nach einem Eingriff am Kniegelenk 5, eingesetzt werden.

Durch den Kompressionsstrumpf 1 wird ein definierter Druck auf das Bein 2 und auf den Kompressionskörper 6 ausgeübt. Figur 2 zeigt eine Rückansicht des Kompressionskörpers 6 nach Fig. 1 in einer vergrößerten und teilweise geschnittenen Darstellung. Der Kompressionskörper 6 weist eine Aussparung 7 zur Aufnahme einer Kniescheibe des Kniegelenks 5 auf. Dadurch wird die Kniescheibe gestützt, geführt und vom Druck des Kompressionskörpers 6 ausgenommen. Sowohl der Kompressionskörper 6, als auch die Aussparung 7 haben in etwa die Form einer Ellipse mit einer Hauptachse 8 und einer Nebenachse 9. Dadurch ist der Kompressionskörper 6 in etwa auf die Form der Gelenkweichteile und die Aussparung 7 in etwa auf die Form der Kniescheibe abgestimmt. Der vom Kompressionsstrumpf 1 aufgebrachte Druck wird über den Kompressionskörper 6 also vollständig und nahezu verlustfrei in die Gelenkweichteile des Kniegelenks 5 eingeleitet. Dadurch wird nach der Operation entstehendes Wundsekret, Blut- und Gewebeflüssigkeit über den venösen Rückfluss aus den Gelenkweichteilen bzw. der Gefäßhülle des Kniegelenks 5 herausgedrückt und Schwellungen des Kniegelenks 5 werden verhindert. Der Kompressionsstrumpf 1 unterstützt dabei diesen venösen Rückfluss der Flüssigkeiten im gesamten Beinbereich und verhindert dadurch eine Thrombose. Im angelegten Zustand des Kompressionsstrumpfes 1 nach Figur 1, ist der Kompressionskörper 6 derart dem Kniegelenk 5 bzw. der Kniescheibe zugeordnet, dass die Hauptachse 8 des Kompressionskörpers 6 in etwa parallel zu einer Körperlängsachse 10 ausgerichtet ist. Zur Aufnahme einer Wundnaht und Wundklammern ist im Kompressionskörper 6 eine nutenförmige Ausnehmung 11 angeordnet. Diese Ausnehmung 11 ist entlang der Hauptachse 8 des Kompressionskörpers 6 auf der gesamten Länge des Kompressionskörpers 6 angeordnet und wird nur durch die Aussparung 7 unterbrochen. Dabei ist die Tiefe der Ausnehmung 11 geringer als die Dicke des Kompressionskörpers 6, was insbesondere der Figur 3 zu entnehmen ist. Der Kompressionskörper 6 kann aus Silikon, aber auch aus jedem anderen geeigneten Material mit ähnlichen Eigenschaften ausgebildet sein.

Durch den erfindungsgemäßen Kompressionsstrumpf 1 mit dem zugeordneten Kompressionskörper 6 wird bei einem postoperativen Einsatz die Wundheilung verbessert, die Schwellung des Knies verhindert bzw. abgebaut und eine Vereiterung verhindert. Außerdem dient der Kompressionsstrumpf der Thromboseprophylaxe.

## Patentansprüche

1. Kompressionsstrumpf (1) aus einem elastischen Gewebe, Gewirke oder Gestricke mit einem an dem Kompressionsstrumpf (1) fixierbaren Kompressionskörper (6) aus einem elastischen Material, welcher eine Aussparung (7) zur Aufnahme einer Kniescheibe eines Kniegelenks (5) aufweist, wobei die benachbarten Gelenkweichteile mittels des die Aussparung (7) einschließenden Kompressionskörpers (6) mit Druck beaufschlagbar sind und eine den Gelenkweichteilen zugewandte Fläche des Kompressionskörpers (6) zumindest eine nutenförmige Ausnehmung (11) aufweist, **dadurch gekennzeichnet, dass** sich die nutenförmige Ausnehmung (11) über eine gesamte Länge des Kompressionskörpers (6) erstreckt und durch die Aussparung (7) unterbrochen ist.

2. Kompressionsstrumpf (1) nach Anspruch 1, **dadurch gekennzeichnet, dass** der Kompressionskörper (6) eine elliptische Form mit einer Hauptachse (8) und einer Nebenachse (9) aufweist.

3. Kompressionsstrumpf (1) nach den Ansprüchen 1 oder 2, **dadurch gekennzeichnet, dass** die nutenförmige Ausnehmung (11) entlang der Hauptachse (8) des Kompressionskörpers (6) angeordnet ist.

4. Kompressionsstrumpf (1) nach zumindest einem der vorhergehenden Ansprüche, **dadurch gekennzeichnet, dass** die nutenförmige Ausnehmung (11) eine Breite von etwa 12 bis 20 mm und eine Tiefe von etwa 4 bis 10 mm aufweist.

5. Kompressionsstrumpf (1) nach zumindest einem der vorhergehenden Ansprüche, **dadurch gekennzeichnet, dass** die Aussparung (7) in dem Kompressionskörper (6) ellipsenförmig ausgebildet ist.

6. Kompressionsstrumpf (1) nach zumindest einem der vorhergehenden Ansprüche, **dadurch gekennzeichnet, dass** die Aussparung (7) bezogen auf eine Hauptachse (8) eine Länge von etwa 70 bis 85 mm und bezogen auf eine Nebenachse (9) eine Breite von etwa 50 bis 60 mm aufweist.

7. Kompressionsstrumpf (1) nach zumindest einem der vorhergehenden Ansprüche, **dadurch gekennzeichnet, dass** der Kompressionskörper (6) mittels einer Adhäsionsverbindung lösbar an dem Kompressionsstrumpf (1) fixiert ist.

8. Kompressionsstrumpf (1) nach zumindest einem der vorhergehenden Ansprüche, **dadurch gekennzeichnet, dass** der Kompressionskörper (6) zumindest abschnittsweise aus Silikon besteht.

9. Kompressionsstrumpf (1) nach zumindest einem der vorhergehenden Ansprüche, **dadurch gekennzeichnet, dass** zwischen dem Kompressionskörper (6) und den Gelenkweichteilen eine Schutzschicht angeordnet ist.

## Claims

1. Compression support stocking (1) made of a knit or woven resilient fabric having a compression body (6) made of a resilient material attachable to the compression support stocking (1), which compression body is provided with a recess (7) for accommodating the patella of a knee joint (5), wherein soft tissue adjacent to the joint can have pressure applied to it by means of the compression body (6) enclosing the recess (7) and wherein a surface of the compression body (6) facing the soft tissue of the joint comprises at least one groove-shaped recess (11), **characterised in that** the groove-shaped recess (11) extends over the entire length of the compression body (6) and is disrupted by the recess (7).

2. Compression support stocking (1) according to claim 1, **characterised in that** the compression body (6) has an elliptical shape with a main axis (8) and a secondary axis (9).

3. Compression support stocking (1) according to claim 1 or 2, **characterised in that** the groove-shaped recess (11) is arranged along the main axis (8) of the compression body (6).

4. Compression support stocking (1) according to at least one of the preceding claims, **characterised in that** the groove-shaped recess (11) is approximately 12 to 20 mm wide and approximately 4 to 10 mm deep.

5. Compression support stocking (1) according to at least one of the preceding claims, **characterised in that** the recess (7) in the compression body (6) has an elliptical configuration.

6. Compression support stocking (1) according to at least one of the preceding claims, **characterised in that** the recess (7) is approximately 70 to 85 mm long based on the main axis (8) and is approximately 50 to 60 mm wide based on the secondary axis (9).

7. Compression support stocking (1) according to at least one of the preceding claims, **characterised in that** the compression body (6) is detachably attached to the compression support stocking (1) by an adhesive connection.

8. Compression support stocking (1) according to at least one of the preceding claims, **characterised in that** the compression body (6) consists at least in part of silicone.

9. Compression support stocking (1) according to at least one of the preceding claims, **characterised in that** a protective layer is arranged between the compression body (6) and the soft tissue of the joint.

## Revendications

1. Bas de contention (1) réalisé dans un tissu, article à mailles ou tricoté élastique, comportant au moins un corps de contention (6) réalisé dans une matière élastique, pouvant être fixé sur le bas de contention (1), lequel comporte un évidement (7) permettant de recevoir la rotule d'une articulation du genou (5), moyennant quoi les parties molles de l'articulation, adjacentes, peuvent être sollicitées par une pression grâce au corps de contention (6) renfermant l'évidement (7), et une surface, orientée vers les parties molles de l'articulation, du corps de contention (6) comporte au moins un creux (11) en forme de rainure, **caractérisé en ce que** le creux (11) en forme de rainure s'étend sur toute la longueur du corps de contention (6), et est interrompu par l'évidement (7).

2. Bas de contention (1) selon la revendication 1, **caractérisé en ce que** le corps de contention (6) se présente sous une forme elliptique, avec un grand axe (8) et un petit axe (9).

3. Bas de contention (1) selon l'une quelconque des revendications 1 ou 2, **caractérisé en ce que** le creux (11) en forme de rainure est ménagé le long du grand axe (8) du corps de contention (6).

4. Bas de contention (1) selon au moins l'une quelconque des revendications précédentes, **caractérisé en ce que** le creux (11) en forme de rainure a une largeur comprise entre environ 12 et 20 mm, et une profondeur comprise entre environ 4 et 10 mm.

5. Bas de contention (1) selon au moins l'une quelconque des revendications précédentes, **caractérisé en ce que** l'évidement (7) dans le corps de contention (6) est configuré sous forme elliptique.

6. Bas de contention (1) selon au moins l'une quelconque des revendications précédentes, **caractérisé en ce que** l'évidement (7), par rapport au grand axe (8), a une longueur comprise entre environ 70 et 85 mm, et, par rapport au petit axe (9), a une largeur comprise entre environ 50 et 60 mm.

7. Bas de contention (1) selon au moins l'une quelconque des revendications précédentes, **caractérisé en ce que** le corps de contention (6) est fixé de manière amovible, à l'aide d'une liaison adhésive, sur le corps de contention (1).

8. Bas de contention (1) selon au moins l'une quelconque des revendications précédentes, **caractérisé en ce que** le corps de contention (6) est constitué, au moins en partie, de silicone.

9. Bas de contention (1) selon au moins l'une quelconque des revendications précédentes, **caractérisé en ce que**, entre le corps de contention (6) et les parties molles de l'articulation, est disposée une couche protectrice.
